# EUROPEAN PATENT APPLICATION

(11) **EP 2 706 395 A2**
(43) Date of publication of application: **12.03.2014**
(21) Application number: 13183361.8
(22) Date of filing: 06.09.2013
(51) Int. Cl.: G02B 27/01

(54) **Sport glasses and use method thereof**

(30) Priority: 11.09.2012 JP 2012199372
(71) Applicant: CASIO COMPUTER CO., LTD., Shibuya-ku, Tokyo 151-8543 (JP)
(72) Inventor: Aibara, Takehiro, Hamura-shi, 205-8555 (JP)
(74) Representative: Grünecker, Kinkeldey, Stockmair & Schwanhäusser

(57) **Abstract**

An exercise support apparatus (100) of the present invention obtains motion information including the pitch and the moving speed of a user based on sensor data detected corresponding to the motion of the user performing an exercise by moving. Subsequently, the exercise support apparatus (100) generates a moving image of a virtual person whose way of movements of feet has been synchronized with the pitch of the user and display size has been set according to a change of the moving speed of the user, based on the obtained motion information. Then, the exercise support apparatus (100) displays the generated moving image on a part of display area arranged in the viewing field of the user, and thereby cause the virtual person to function as a pacemaker for the user.

## Description

### BACKGROUND OF THE INVENTION

### 1. Field of the Invention

The present invention relates to an exercise support apparatus and an exercise support method. Specifically, the present invention relates to an exercise support apparatus and an exercise support method that can be applied to exercises such as walking or running.

### 2. Description of the Related Art

In recent years, because of rising health consciousness, more and more people are performing daily exercises, such as running, walking, and cycling, to maintain their wellness or improve their health condition. In addition, an increasing number of people are aiming to participate in a race such as a marathon race through these daily exercises. These people aiming to participate in a race have an objective of achieving a successful record in the race, and therefore are very conscious of and interested in efficient and effective training methods.

Devices for fulfilling these demands have been variously developed as of now. For example, technologies have been developed in which various information regarding a performed exercise (for example, a running time, a current rank in a competition, and vital information such as a heart rate) are displayed and provided to the user via a wristwatch-type information terminal mounted on a wrist or a head-mount display mounted on the head. Here, an example of a technology for providing various information via a head-mount display is described in Japanese Patent Application Laid-Open (Kokai) Publication No. 2008-099834.

In practices for running or running in a marathon, there is a training method in which a really-existing pacemaker actually runs before a runner to keep or adjust the rhythm of running of the following runner and to slowly increase his or her running speed, where by a better record is achieved. This training method using a pacemaker can achieve a high exercise effect, and an excellent record can be expected. Accordingly, the pacemaker plays an important role. However, in view of securing human resources and also from an economic point of view, it is very difficult to find a pacemaker capable of appropriately guiding a runner with the understanding of his or her proficiency.

Among the devices such as the information terminal and the head-mount display described above, a device that provides information serving as a pacemaker to a user has been known.

However, the information provided by this device is, for example, merely numerical value information such as a pitch (a footstep count) or a running speed. In addition, all it does to display the numerical value information as a pacemaker is to change the display format or the display method.

That is, conventional technologies have not yet achieved a function for drawing the following runner, or in other words, a function for guiding the user to an appropriate running state to achieve a high exercise effect and an excellent record, as with a really-existing pacemaker

### SUMMARY OF THE INVENTION

The present invention can advantageously provide an exercise support apparatus and an exercise support method that contributes to achievement of a high exercise effect and an excellent record in an exercise such as moving, by appropriately guiding a user like an actual pacemaker.

In accordance with one aspect of the present invention, there is provided an exercise support apparatus comprising: a sensor section which outputs motion data corresponding to a motion status of a user performing an exercise by moving; a motion information obtaining section which obtains motion information of the user based on the motion data; an image generating section which generates a moving image of a virtual person in a moving state, and sets a way of movements of feet of the virtual person to a way of movements corresponding to the obtained motion information of the user; and a display section which displays the moving image on a part of a display area arranged in a viewing field of the user.

In accordance with another aspect of the present invention, there is provided an exercise support method for an exercise support apparatus including a display section having a display area that is arranged in a viewing field of a user, comprising: a step of obtaining motion information of the user based on motion data corresponding to a motion status of the user performing an exercise by moving; a step of generating a moving image of a virtual person in a moving state; a step of setting a way of movements of feet of the virtual person to a way of movements corresponding to the obtained motion information of the user; and a step of displaying the moving image on a part of the display area of the display section.

The above and further objects and novel features of the present invention will more fully appear from the following detailed description when the same is read in conjunction with the accompanying drawings. It is to be expressly understood, however, that the drawings are for the purpose of illustration only and are not intended as a definition of the limits of the invention.

### BRIEF DESCRIPTION OF THE DRAWINGS

FIG. 1A, FIG. 1B, and FIG. 1C are schematic structural views of an exercise support apparatus according to a first embodiment of the present invention;
FIG. 2 is a block diagram showing an example of structure of display glasses applied to the exercise support apparatus according to the first embodiment;
FIG. 3A, FIG. 3B, and FIG. 3C are schematic diagrams each depicting an example of a method of displaying a virtual person applied in an exercise support method according to the first embodiment;
FIG. 4 is a flowchart depicting a first example of a normal mode applied to the exercise support method according to the first embodiment;
FIG. 5 is a flowchart depicting a second example of a normal mode applied to the exercise support method according to the first embodiment;
FIG. 6 is a flowchart of an example depicting a long-distance running mode applied to the exercise support method according to the first embodiment;
FIG. 7 is a flowchart of an example of a pace set mode applied to the exercise support method according to the first embodiment;
FIG. 8 is a flowchart of an example of a build-up mode applied to the exercise support method according to the first embodiment;
FIG. 9A, FIG. 9B, and FIG. 9C are schematic structural views of an exercise support apparatus according to a second embodiment of the present invention;
FIG. 10A and FIG 10B are block diagrams showing an example of structure of a device applied to the exercise support apparatus according to the second embodiment; and
FIG. 11A and FIG 11B are block diagrams showing an example of structure of a device applied to an exercise support apparatus according to a third embodiment.

### DETAILED DESCRIPTION OF THE PREFERRED EMBODIMENTS

An exercise support apparatus and an exercise support method according to embodiments of the present invention are described in detail below.

In the following description, as an example of the exercise, the case is described where a user conducts training by running a marathon. However, the present invention is not limited thereto, and can be applied to any other exercise such as walking.

### <First Embodiment>

### (Exercise Support Apparatus)

First, an exercise support apparatus according to the present invention is described.

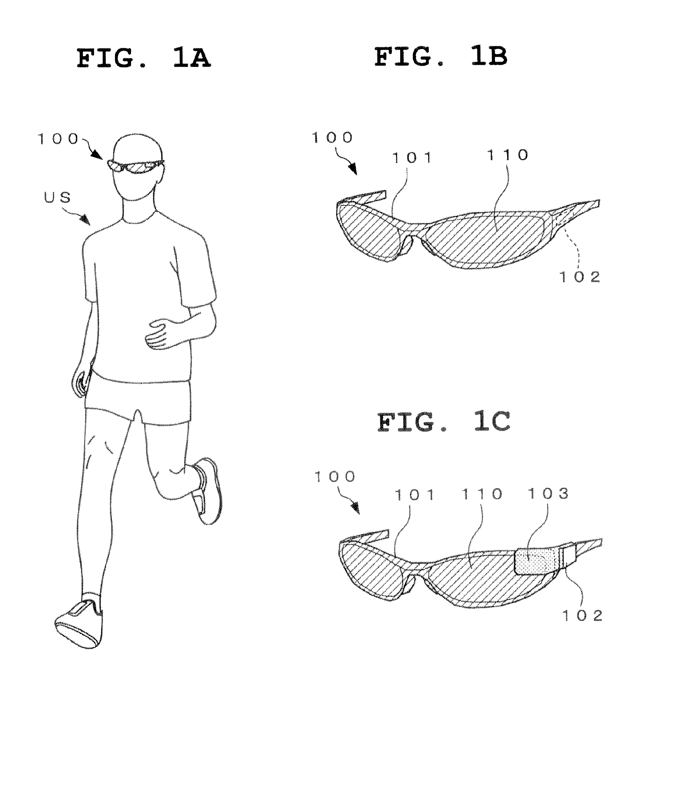

FIG. 1A, FIG. 1B, and FIG. 1C are schematic structural views of an exercise support apparatus according to a first embodiment of the present invention.

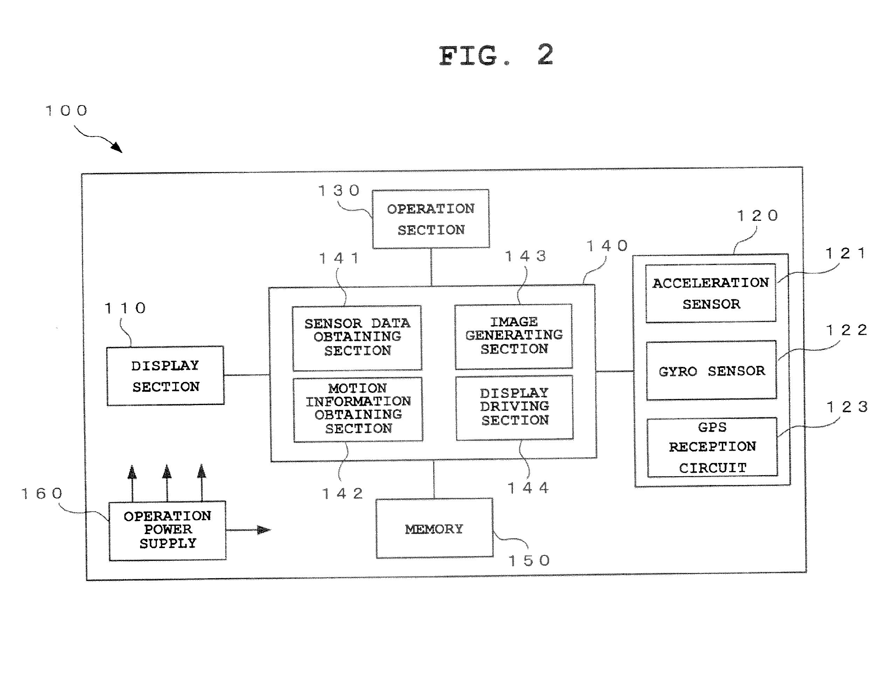

FIG. 2 is a block diagram of an example of structure of display glasses applied to the exercise support apparatus according to the first embodiment.

The exercise support apparatus according to the present embodiment has, for example, display glasses (a head-mount display) 100 mounted on the head part of a user US who is a runner, as schematically depicted in FIG. 1A.

The display glasses 100 have an outer appearance of, for example, eyeglasses-type or a goggles-type, as depicted in FIG. 1B and FIG. 1C.

The display glasses 100 mainly includes a main body 101 having a transparent-type display section 110 which is positioned in an area immediately in front of the eyes of the user US and in which the lenses of eyeglasses or goggles are positioned, and temples for mounting the display glasses 100 on the head part; and a display control section 102 which performs transparent display or projection display of a desired image and various exercise information on a part of the display section 110 of the main body 101 to visually provide the image or information to the user US.

Here, the display glasses 100 may have a structure in which the display control section 102 is integrally provided inside the main body 101, as depicted in FIG. 1B, or a structure in which the display control section 102 including an image projecting apparatus 103 is additionally assembled on commercially-available sports glasses, sunglasses or eyeglasses, as depicted in FIG. 1C.

Specifically, the display glasses 100 mainly includes, for example, the display section 110, a motion sensor section 120, an operation section 130, a central computation circuit (hereinafter referred to as a "CPU") 140, a memory 150, and an operation power supply 160, as depicted in FIG. 2.

Here, the display control section 102 is provided with components of the display glasses 100 other than the display section 110, that is, the motion sensor section 120, the operation section 130, the CPU 140, the memory 150, and the operation power supply 160.

In the structure where the display control section 102 is integrally provided in the main body 101 as depicted in FIG. 1B, for example, the display section 110 can have a transparent-type display panel in place of the lens of the eyeglasses or goggles. As this display panel, for example, a transparent-type liquid-crystal display panel or an organic EL display panel capable of color or monochrome display can be applied. As a result, a desired image and exercise information are transparently displayed in the viewing field of the user US in a manner to be superimposed on the surrounding view.

On the other hand, in the structure where the display control section 102 is additionally assembled on commercially-available sports glasses or the like as depicted in FIG. 1C, the display section 110 is structured to perform transparent display directly onto the image projection apparatus 103 or to perform projection display on a transparent glass, transparent resin, or the like of a lens of the sports glasses immediately in front of the eyes of the user US. In this case as well, a desired image and exercise information are displayed in the viewing field of the user US in a manner to be superimposed on the surrounding view.

Here, in the present embodiment, as an image displayed on a part of the display section 110, a moving image or a still image of a virtual person serving as a pacemaker dedicated for the user US is displayed, which is generated based on the exercise support method described further below.

On the display section 110, in addition to the image of the virtual person, for example, numerical information and character information regarding the exercise performed by the user US (for example, pitch (footstep count), running speed, run distance, and calorie consumption amount) are displayed as exercise information.

These image and exercise information may be displayed simultaneously on the display section 110, or either one of the image and one or plurality of exercise information may be displayed by operating the operation section 130, which will be described further below.

The motion sensor section 120 has an acceleration sensor 121, a gyro sensor (angular velocity sensor) 122, and a GPS (Global Positioning System) reception circuit 123, for example, as depicted in FIG. 2.

The acceleration sensor 121 detects an acceleration corresponding to the change ratio of the motion speed of the user US during running, and outputs acceleration data corresponding to the acceleration. Then, based on this acceleration data outputted from the acceleration sensor 121, relative changes of the pitch (footstep count per second) and the running speed (pace) of the user US are obtained.

The gyro sensor (angular velocity sensor) 122 detects an angular velocity corresponding to a change in the moving direction of the user US during an exercise and outputs angular velocity data corresponding to the angular velocity. Then, based on this angular velocity data outputted from the gyro sensor 122 and a change tendency of the acceleration data and a waveform peak frequency outputted from the acceleration sensor 121 described above, absolute values of the pitch and the running speed at the time of running are obtained.

Furthermore, based on the above-described running speed and an elapsed time, a run distance is obtained.

The GPS reception circuit 123 is a position sensor which receives electric waves from a plurality of GPS satellites to detect a (geographical) position composed of latitude and longitude and outputs position data corresponding to the position. Based on the position data outputted from the GPS reception circuit 123, the GPS reception circuit 123 can obtain the moving distance (that is, run distance) of the user US.

Here, the acceleration data outputted from the acceleration sensor 121, the angular velocity data outputted from the gyro sensor 122, and the position data outputted from the GPS reception circuit 123 are collectively referred to as sensor data (motion data).

As such, the motion sensor section 120 according to the present embodiment has at least the acceleration sensor 121 and the gyro sensor 122 depicted in FIG. 2, or a pressure sensor and the like for another structure, as a sensor for obtaining the pitch of the user US.

Also, the motion sensor section 120 has at least one of the sensor group constituted by the acceleration sensor 121 and the gyro sensor 122 depicted in FIG. 2 and the GPS reception circuit 123, as a sensor for obtaining the running speed of the user US.

That is, the motion sensor section 120 is required to include the acceleration sensor 121 and the gyro sensor 122 described above, and may not be structured to include the GPS reception circuit 123.

The moving distance data and the moving speed data obtained based on the position data outputted from the GPS reception circuit 123 may be used together or complementarily with the run distance and the running speed obtained based on the acceleration data and the angular velocity data outputted from the acceleration sensor 121 and the gyro sensor 122 described above so as to increase the accuracy of the run distance and the running speed of the user US.

These pitch, running speed, and run distance are measured or calculated based on the sensor data from each of the above-described sensors by the CPU 140 described below executing a predetermined program.

These pitch, running speed, and run distance are then associated with each other for each running time, and are each stored in a predetermined storage area of the memory 150 described below.

The operation section 130 has at least a power supply switch, and controls supply (power supply ON) and shutoff (power supply OFF) of driving power from the operation power supply 160 described below to each component inside the display glasses 100.

The operation section 130 is used for setting display of the exercise information on the display section 110 described above, selection of a motion (training) mode in the exercise support method described below, selection of an image design (for example, body-build, gender, or costume) of the virtual person in the motion mode, inputs of numerical value conditions, a display position of the virtual person on the display section 110 (for example, whether to display the virtual person in a left viewing field or a right viewing field), a pause of the motion of the virtual person, etc.

The memory 150 has a non-volatile memory, and stores the acceleration data and the angular velocity data outputted from the motion sensor section 120 described above, the sensor data such as the position data, and the motion information including the pitch, the running speed, the run distance, etc. at the time of running obtained based on these sensor data, in association with each other for each running time.

In the non-volatile memory part of the memory 150, various data and information generated or referred to by the exercise support method described below are stored.

Here, the memory 150 may include a Read Only Memory (ROM) having stored therein control programs (software) for achieving predetermined functions of the display section 110, the motion sensor section 120, the CPU 140, and the memory 150.

The non-volatile memory part forming the memory 150 may have a removable storage medium such as a memory card, and may be structured to be removable from the display glasses 100.

The CPU 140 has a clock function and performs processing by following a predetermined program to control the operations of the display section 110, the motion sensor section 120, and the memory 150 and achieve predetermined functions. The control program may be stored in the memory 150 described above or may be incorporated in advance in the CPU 140.

Specifically, the CPU 140 mainly includes a sensor data obtaining section 141, a motion information obtaining section 142, an image generating section 143, and a display driving section 144, as depicted in FIG. 2.

The sensor data obtaining section 141 obtains acceleration data of the user US during running from the acceleration sensor 121 of the motion sensor section 120 described above.

The motion information obtaining section 142 detects the pitch of the user US, and landing and takeoff (landing/takeoff) timing of the feet of the user US based on the acceleration data obtained by the sensor data obtaining section 141.

The image generating section 143 generates a moving image of the virtual person based on the pitch and the landing/takeoff timing obtained by the motion information obtaining section 142.

That is, the image generating section 143 sets a replay speed, a replay method, a display size, etc., of the moving image of the virtual person such that they correspond to the obtained pitch and landing/takeoff timing.

The display driving section 144 causes the image of the virtual person generated by the image generating section 143 to be displayed on a partial area of the display section 110 of the display glasses 100.

The exercise support method that is achieved by the CPU 140 will be described in detail further below.

The operation power supply 160 supplies driving electric power to each components of the display glasses 100. As the operation power supply 160, for example, a primary battery such as a commercially-available coin-shaped battery or button-shaped battery or a secondary battery such as a lithium-ion battery or a nickel-metal-hydride battery can be applied. In addition, it is possible to apply a power supply by energy harvest technology for generating electricity by energy such as vibrations, light, heat, or electro-magnetic waves.

### (Exercise Support Method; Virtual Person Display Method)

Next, the exercise support method in the exercise support apparatus according to the present embodiment is described.

First, a virtual person display method applied to the exercise support method according to the present invention is described.

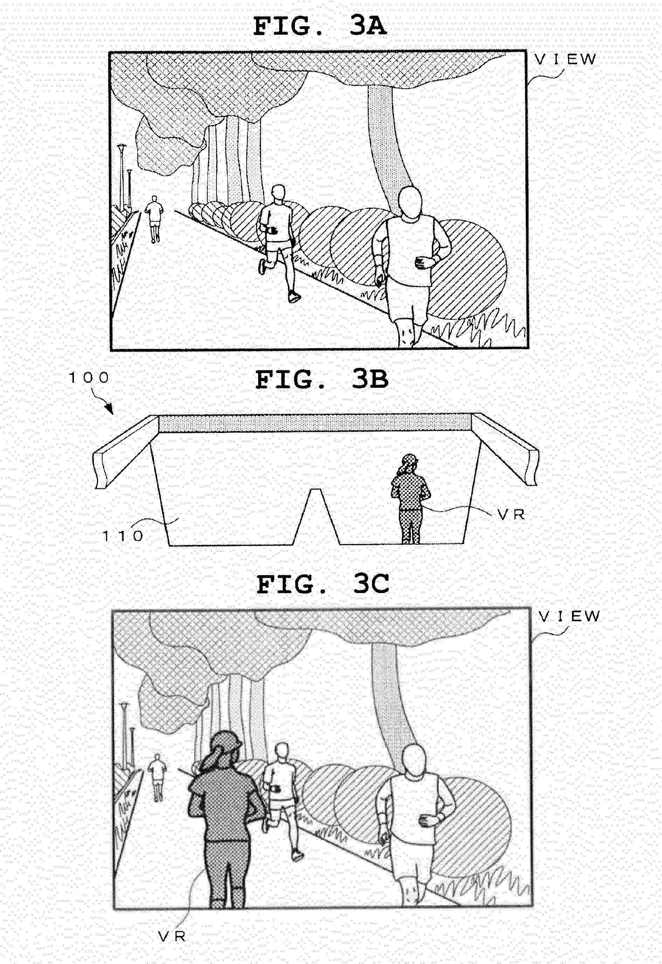

FIG. 3A, FIG. 3B, and FIG. 3C are schematic diagrams each depicting an example of the method of displaying the virtual person applied in the exercise support method according to the present embodiment.

Here, the case is described where the user US is running as a runner in a view VIEW as depicted in FIG. 3A.

In the virtual person display method applied in the exercise support method according to the present embodiment, in the viewing field of the user US wearing the display glasses 100 described above, the actual view VIEW surrounding the user US as depicted in FIG. 3A is first recognized through the transparent-type display panel or transparent glasses of the display section 110.

Then, on the display glasses 100, by performing the exercise support method described below, the motion mode set by the user US and a moving image or a still image of a virtual person VR serving as a pacemaker dedicated for the user US, which is generated based on the current exercise status of the user US, are displayed in a predetermined display format in a predetermined partial area of the display section 110.

Here, the exercise support apparatus according to the present embodiment is constituted by the display glasses 100 that is a single device, and the display section 110 has a transparent display area such as a transparent-type display panel or a transparent glass.

Thus, as depicted in FIG. 1A, the surrounding view VIEW can be viewed by the user US through the display section 110 with a simple head-mounting method. Also, the virtual person VR is displayed on a part of the display area.

That is, in the viewing field of the user US, the view VIEW transmitted through the display section 110 and the image of the virtual person VR displayed on the display section 110 are viewed in a manner to be superposed with each other, as depicted in FIG. 3C. As a result, the user US recognizes as if the virtual person VR serving as the pacemaker dedicated for the user US is running in front of the user US in the front view VIEW.

In particular, in the virtual person display method applied to the present embodiment, a display state is used as a reference in which the virtual person VR displayed on the display section 110 is running with the same pitch as that of the user US who is a runner so as to keep a predetermined positional relation (clearance) with the user US. If the running speed of the user US has reduced with reference to this state, the display state is changed such that the virtual person VR moves (proceed) toward the front and positioned away from the user US.

Accordingly, the user US is caused to recognize that the virtual person VR is moving away from the user US and become aware that he or she needs to increase the pace to catch up with the virtual person VR, and thereby prompted to increase the pace.

As such, according to the virtual person display method applied to the present embodiment, as with an actual pacemaker, the user US can be drawn (guided) by the virtual person VR to run fast, and thereby supported (assisted) to improve his or her physical capability.

In a specific example of the exercise support method described further below, a state where the virtual person VR displayed on the display section 110 is changed, how it is changed, and how it is returned to its original state (an initial state) are described by specifically presenting various motion modes (training modes) set in the display glasses 100.

Note that the image of the virtual person VR, which is displayed on the display section 110 of the display glasses 100, is not particularly limited to that shown in FIG. 3B to FIG. 3C. For example, the image design can be changed or another image design can be selected by the user US operating the operation section 130 or by one of the motion modes in the exercise support method described below being selected and set (that is, by a program).

Also, in FIG. 3B, the case is depicted where the image of the virtual person is displayed on the right side or the left side (one side) of the viewing field of the display section 110 of the display glasses 100. However, a configuration may be adopted in which, by operating this display position with the operation section 130, the user US can select whether the image of the virtual person is displayed on the right side of the viewing field or the left side of the viewing field, and can move and adjust the display position in any of the leftward, rightward, upward and downward directions in the display section 110.

### (Exercise Support Method; Specific Example in Various Motion Modes)

Next, the exercise support method to which the above-described virtual person display method has been applied is described by specifically presenting various motion modes (training modes).

The display glasses applied to the exercise support apparatus according to the present embodiment have a plurality of motion modes (training modes), and one of the motion modes is selected and set by the user US operating the operation section 130.

Then, according to this selected motion mode, a replay speed, a replay method, a display format such as the display size of the image of the virtual person VR displayed on the display section 110 described above are individually set. A series of operations according to the exercise support method is achieved by the CPU 140 of the display glasses 100 described above by following a predetermined control program.

Here, in the exercise support method according to the present embodiment, in a case where the motion sensor section 120 of the display glasses 100 described above has only the acceleration sensor 121, a normal mode and a long-distance running mode can be selected, set, and performed, which will be described further below.

On the other hand, in a case where the motion sensor section 120 has at least the acceleration sensor 121 and the gyro sensor 122, all motion modes described below can be selected, set, and performed.

### (First Example of Normal Mode)

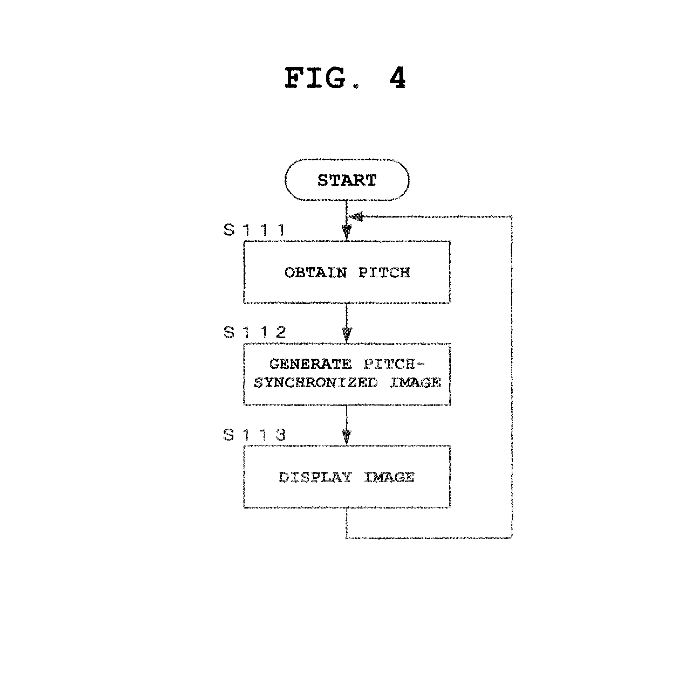

FIG. 4 is a flowchart of a first example of a normal mode applied to the exercise support method according to the present embodiment.

The normal mode applied to the present embodiment is achieved by the user US operating the operation section 130 of the display glasses 100 mounted on the head part to select and set the normal mode as a motion mode, whereby the CPU 140 calls a program module of the control program regarding the normal mode to perform processing.

In the first example of the normal mode, the CPU 140 first starts an operation of controlling the motion sensor section 120 to detect at least the acceleration of the user US during an exercise (during running) by the acceleration sensor 121 and output acceleration data.

Next, the CPU 140 causes the sensor data obtaining section 141 to obtain the acceleration data from the acceleration sensor 121 and causes the motion information obtaining section 142 to obtain motion information such as the pitch and the landing/takeoff timing of the feet of the user US based on the acceleration data, as depicted in FIG. 4 (Step S111).

Next, the CPU 140 causes the image generating section 143 to match the landing/takeoff timing of the feet of the virtual person with the current landing/takeoff timing of the feet of the user US based on the obtained pitch and landing/takeoff timing of the feet and also match the pitch of the virtual person VR with the current pitch of the user US. As a result, a moving image (a pitch-synchronized image) of the virtual person VR in synchronization with the motion status of the user US is generated (Step S112).

Next, the CPU 140 causes the display driving section 144 to cause the generated moving image of the virtual person VR to be displayed in a predetermined area of the display section 110 (Step S113).

The CPU 140 then performs this series of processing at predetermined time intervals or repeatedly at all times.

### (Second Example of Normal Mode)

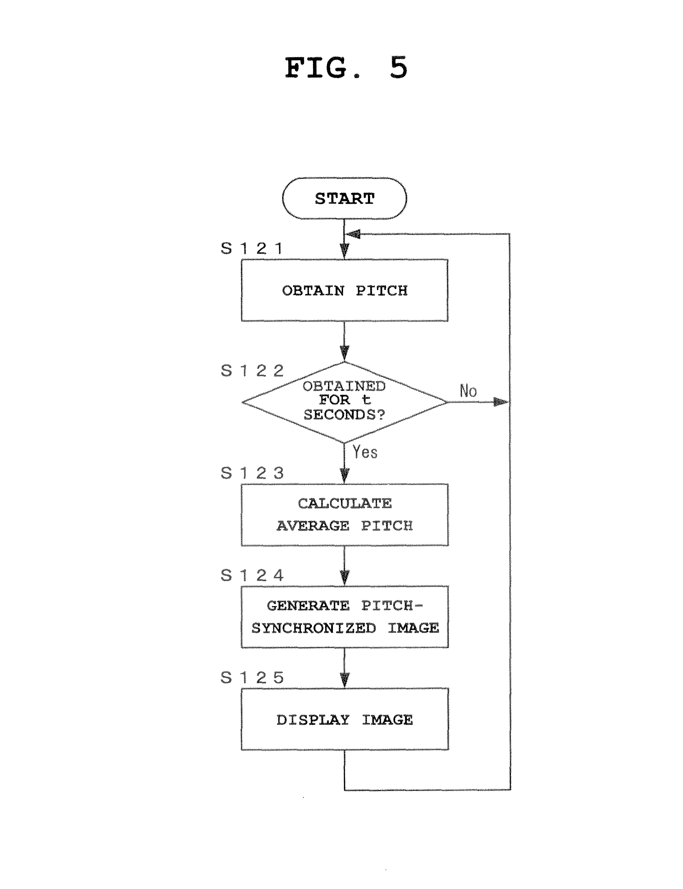

FIG. 5 is a flowchart depicting a second example of the normal mode applied to the exercise support method according to the present embodiment.

Here, description of processing operations similar to those of the first example of the normal mode described above is simplified.

The pitch and the landing/takeoff timing of the feet of the user US may temporally vary depending on, for example, the status of the surrounding runners or the running course. In the second example of the normal mode, the moving image of the virtual person VR is set based on average values of pitches and landing/takeoff timings of the feet of the user US in a predetermined short period of time.

In the second example of the normal mode, the CPU 140 first starts an operation of causing the motion sensor section 120 (the acceleration sensor 121) to detect the acceleration of the user US during an exercise and output acceleration data.

Next, the CPU 140 causes the sensor data obtaining section 141 and the motion information obtaining section 142 to obtain motion information such as the pitch of the user US and the landing/takeoff timing of the feet of the user US based on the acceleration data obtained from the acceleration sensor 121, as depicted in FIG. 5 (Step S121).

Next, the CPU 140 causes the obtainment of the pitch and the landing/takeoff timing of the feet described above to continue for t second (for example, ten seconds), and thereby retains the motion information (Step S122).

Next, the CPU 140 causes the motion information obtaining section 142 to calculate an average value of the pitches (an average pitch) and an average value of the landing/takeoff timings of the feet (an average timing) based on the obtained pitches and landing/takeoff timings of the feet of the user US obtained continuously for ten seconds (Step S123).

At Step S122 and Step S123, a time for obtaining the pitch and the landing/takeoff timing of the feet of the user US (a sampling time) is set at ten seconds. However, the present invention is not limited thereto. For example, any sampling time, such as five seconds or thirty seconds, may be set. Here, if the sampling time is set too long, a discrepancy between the actual pitch of the user US and the pitch of the virtual person VR, which will be described further below, may be large (these pitches may become separated from each other). Therefore, a relatively short time, such as five seconds or ten seconds, should preferably be set.

Next, based on the calculated average pitch and average timing, the CPU 140 causes the image generating section 143 to match the landing/takeoff timing of the feet of the virtual person VR virtual person VR with the average timing and also match the pitch of the virtual person VR with the average pitch. As a result, a moving image (a pitch-synchronized image) of the virtual person VR in synchronization with an average motion status of a user US between sampling times is generated (Step S124).

Next, the CPU 140 causes the display driving section 144 to cause the generated moving image of the virtual person VR to be displayed in a predetermined area of the display section 110 (Step S125). The CPU 140 then performs the series of processing at predetermined time intervals or repeatedly at all times.

As a result, in each normal mode described above (the first and second normal modes), the user US who is a runner can recognize a moving image of the pacemaker dedicated for the user US (virtual person VR) which is running in front of the user US with the current (real-time) pitch or the immediately preceding (for example, an average for previous ten seconds) pitch.

Therefore, the user US continues running so as to follow the back (image) of the virtual person VR running ahead, and thereby can be drawn (the running can be guided) by the virtual person VR to contribute to an improvement of the physical capability of the user US (that is, to achieve a high exercise effect and an excellent record).

In the normal modes described above, the processing operation to which only the acceleration data outputted from the acceleration sensor 121 is applied is described. However, the present invention is not limited thereto.

That is, in the normal modes described above, in addition to the acceleration data, angular velocity data or position data outputted from the gyro sensor 122 and the GPS reception circuit 123 of the motion sensor section 120 may be applied, and accordingly reflected onto the image and the exercise information displayed on the display section 110.

### (Long-Distance Running Mode)

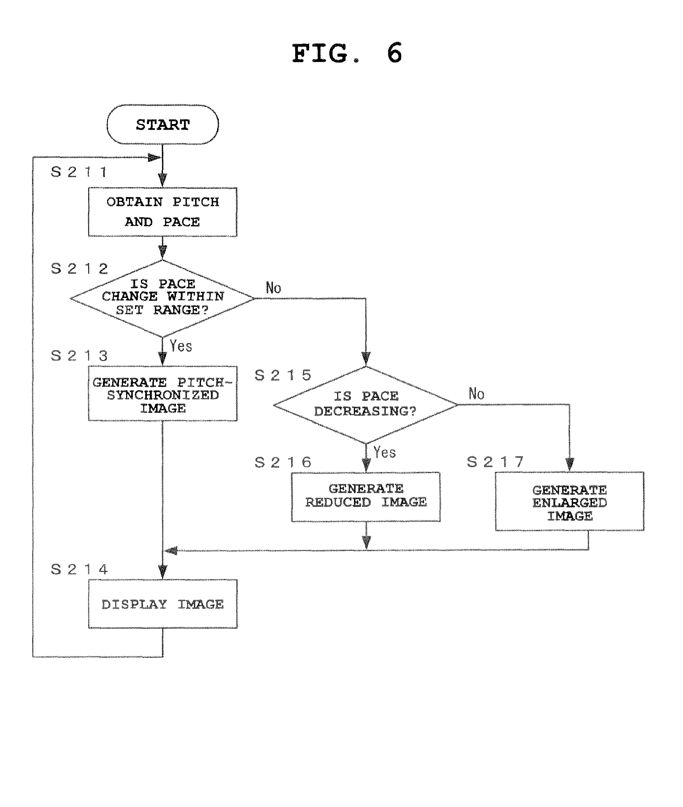

FIG. 6 is a flowchart of an example depicting a long-distance running mode applied to the exercise support method according to the present embodiment.

Here, description of processing operations similar to those of the normal modes described above is simplified.

The long-distance running mode is a motion mode in which large fluctuations in running speed are suppressed by the virtual person VR to prompt the user to run at an approximately constant speed.

The long-distance running mode applied to the present embodiment is achieved by the user US operating the operation section 130 of the display glasses 100 to select and set the long-distance running mode as a motion mode, whereby the CPU 140 calls a program module of the control program regarding the long-distance running mode to perform processing.

In the long-distance running mode, the CPU 140 first starts an operation of controlling the motion sensor section 120 to detect the acceleration of the user US during an exercise (during running) by the acceleration sensor 121 and output acceleration data.

Next, the CPU 140 causes the sensor data obtaining section 141 to obtain the acceleration data from the acceleration sensor 121, and then causes the motion information obtaining section 142 to obtain motion information such as the pitch and the landing/takeoff timing of the feet of the user US based on the acceleration data and a change of the running speed (pace), as depicted in FIG. 6 (Step S211).

Next, the CPU 140 judges whether the obtained change of the running speed is within a range set in advance (Step S212).

When judged at Step S212 that the change of the running speed is within the set range, based on the obtained pitch and landing/takeoff timing of the feet, the CPU 140 causes the image generating section 143 to generate a moving image (a pitch-synchronized image) of the virtual person VR in synchronization with the motion status of the user US by matching the landing/takeoff timing of the feet of the virtual person VR with the current landing/takeoff timing of the feet of the user US and matching the pitch of the virtual person VR with the current pitch of the user US (Step S213).

Next, the CPU 140 causes the display driving section 144 to cause the moving image of the virtual person VR generated so as to be synchronized with the motion status of the user US to be displayed in a predetermined area of the display section 110 (Step S214).

On the other hand, when judged at Step S212 that the change of the running speed is not within the set range, based on the change of the running speed, the CPU 140 judges whether the running speed of the user US is decreasing (Step S215).

When judged at Step S215 that the running speed of the user US is significantly deceasing (for example, equal to or more than a threshold), the CPU 140 causes the image generating section 143 to generate the moving image of the virtual person VR with its display size reduced more than the display size at the previous Step S214, according to the degree of the change

(degrease) of the running speed (Step S216). That is, as the degree of the decrease of the running speed becomes larger, the display size becomes relatively smaller.

Next, the CPU 140 causes the display driving section 144 to cause the moving image of the virtual person VR generated by reducing the display size according to the decrease of the running speed of the user US to be displayed in a predetermined area of the display section 110 (Step S214).

On the other hand, when judged at Step S215 that the running speed of the user US is not decreasing, the CPU 140 judges that the running speed of the user US is increasing. Then, the CPU 140 causes the image generating section 143 to generate a moving image of the virtual person VR with its display size enlarged more than the display size at the previous Step S214, according to the degree of the change (increase) of the running speed (Step S217). That is, as the degree of the increase of the running speed becomes larger, the display size becomes relatively larger.

Next, the CPU 140 causes the display driving section 144 to cause the moving image of the virtual person VR generated by enlarging the display size according to the increase of the running speed of the user US to be displayed in a predetermined area of the display section 110 (Step S214).

The CPU 140 then performs the series of processing at predetermined time intervals or repeatedly at all times.

As a result, when the running speed (pace) of the user US is relatively decreasing, the virtual person VR is displayed smaller, and therefore the user US recognizes that the virtual person VR has moved away ahead. Here, the user US notices that he or she is not keeping pace with the running of the virtual person VR and is in a delay state. As a result, the user US is prompted to catch up with the virtual person VR running ahead by increasing the pitch or extending his or her stride (footstep width) to increase the pace.

On the other hand, when his or her running speed is relatively increasing, the virtual person VR is displayed larger, and therefore the user US recognizes that he or she is approaching the virtual person VR. Here, the user US notices that he or she is starting to keep pace with the virtual person VR if lagging behind the virtual person VR.

If not lagging behind the virtual person VR, the user US notices that he or she is running faster than the virtual person VR and is in an over pace state.

As a result, the user US can be drawn (the running can be guided) by the virtual person VR so as to suppress large fluctuations of the running speed, which contributes to an improvement of the physical capability of the user US.

### (Pace-Set Mode)

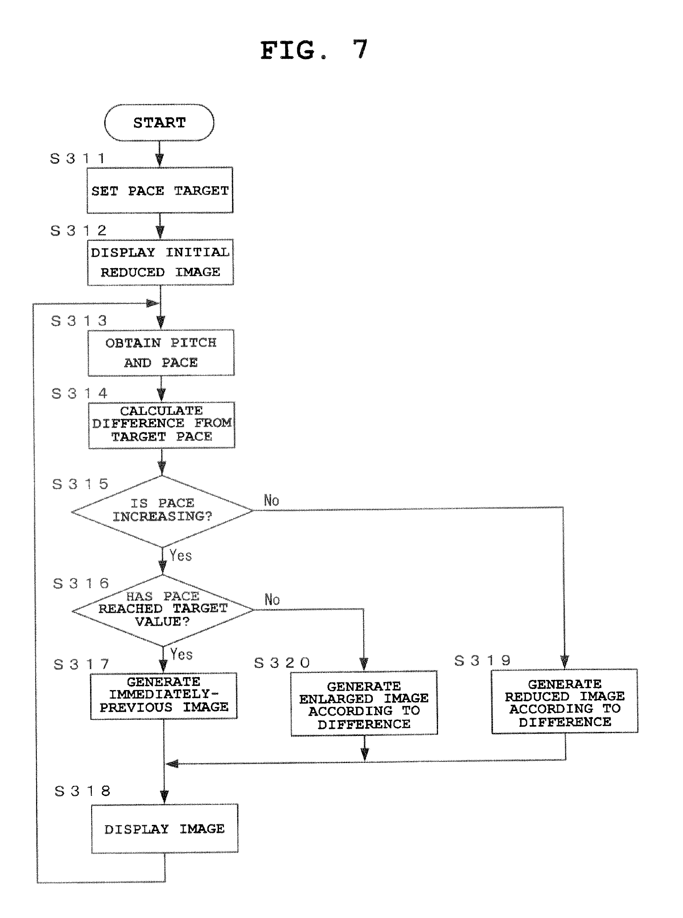

FIG. 7 is a flowchart of an example depicting a pace set mode applied to the exercise support method according to the present embodiment.

Here, description of processing operations similar to those of the normal modes and the long-distance running mode described above is simplified.

The pace-set mode is a motion mode in which the running speed is promoted to be brought closer to a preset target value by the virtual person VR.

The pace-set mode applied to the present embodiment is achieved by the user US operating the operation section 130 of the display glasses 100 to select and set the pace-set mode as a motion mode, whereby the CPU 140 calls a program module of the control program regarding the pace-set mode to perform processing.

In the pace-set mode, the user US first operates the operation section 130 of the display glasses 100 to input and set a target value of the running speed (pace), as depicted in FIG. 7 (Step S311).

Next, the CPU 140 causes the image generating section 143 and the display driving section 144 to cause a moving image of the virtual person VR registered in advance as an initial image to be displayed in a predetermined area of the display section 110 (Step S312).

Here, the initial image is generated as a moving image whose display size has been reduced so as to achieve a state where the virtual person VR is running in the front of the viewing field of the user US in an area relatively away from the user US.

Next, the CPU 140 starts an operation of controlling the motion sensor section 120 to detect at least the acceleration and the angular velocity of the user US during an exercise (during running) by the acceleration sensor 121 and the gyro sensor 122 and output acceleration data and angular velocity data.

Next, the CPU 140 causes the sensor data obtaining section 141 to obtain the acceleration data from the acceleration sensor 121 and the angular velocity data from the gyro sensor 122.

The CPU 140 then causes the motion information obtaining section 142 to obtain motion information such as the pitch and the landing/takeoff timing of the feet of the user US based on the acceleration data and the angular velocity data, and an absolute value of the running speed (pace) (Step S313).

Next, the CPU 140 calculates a difference between the obtained running supped and the target value (target pace) of the running speed set in advance (the obtained value-the target value) (Step S314).

The CPU 140 then judges based on the calculated difference whether the obtained running speed is increasing (whether an absolute value of the difference is reducing) (Step S315).

When judged at Step S315 that the running speed is increasing (the absolute value of the difference is reducing), the CPU 140 judges whether the running speed has reached the target value (Step S316).

When judged at Step S316 that the running speed has reached the target value, the CPU 140 causes the image generating section 143 to enlarge the display size to a standard size set in advance so as to achieve a state where the virtual person VR is running in an area immediately in front of the user US in the viewing field, match the landing/takeoff timing of the feet of the virtual person VR with the landing/takeoff timing of the feet of the user US and the pitch of the virtual person VR with the pitch of the user US based on the pitch and the landing/takeoff timing of the feet of the user US, and generate a moving image (a standard image) of the virtual person VR in synchronization with the motion status of the user US (Step S317).

Next, the CPU 140 causes the display driving section 144 to enlarge the display size and to cause the moving image of the virtual person VR generated in synchronization with the motion status of the user US to be displayed in a predetermined area of the display section 110 (Step S318).

On the other hand, when judged at Step 315 that the running speed is not increasing (the absolute value of the difference is increasing), the CPU 140 judges that the running speed of the user US is decreasing. The CPU 140 then causes the image generating section 143 to generate a moving image of the virtual person VR whose display size has been reduced in, for example, inverse proportion to the size of the initial image, according to the magnitude of the absolute value of the difference (Step S319).

Next, the CPU 140 causes the display driving section 144 to cause the moving image of the virtual person VR generated by being reduced according to the decrease of the running speed to be displayed in a predetermined area of the display section 110 (Step S318).

When judged at Step S316 that the running speed has not reached the target value, the CPU 140 causes the image generating section 143 to generate a moving image of the virtual person VR with its display size enlarged in, for example, inverse proportion to the size of the initial image, according to the magnitude of the absolute value of the difference (Step S320).

Next, the CPU 140 causes the display driving section 144 to cause the moving image of the virtual person VR generated by being enlarged according to the increase of the running speed to be displayed in a predetermined area of the display section 110 (Step S318).

The CPU 140 then performs this series of processing at predetermined time intervals or repeatedly at all times.

As a result, the display size of the virtual person VR displayed in the front of the viewing field of the user US at the time of the start of the running is set to be small, whereby the user US recognizes that the virtual person VR is running in front of the user US in an area relatively away from the user US. Then, as his or her running speed (pace) becomes closer to the target value set in advance, the display size of the virtual person VR is set larger. Consequently, the user US gradually catches up with the virtual person VR.

That is, by adjusting the running speed (pace) so that the distance from the virtual person VR running ahead is kept constant, the user US can be drawn (the running is guided) by the virtual person VR at the running speed of the target value set in advance, which contributes to an improvement of the physical capability of the user US.

In the pace-set mode described above, a processing operation is described in which the user US sets a running speed (pace) that serves as a target value in advance and achieves running that is approximate to this target value. However, the present invention is not limited thereto.

That is, in the pace-set mode described above, control may be performed by which, in addition to the running speed (pace), an arbitrary pitch is set as a target value and this pitch is increased and decreased to correspond to the display size (that is, a distance from the user US) of the virtual person VR.

### (Build-Up Mode)

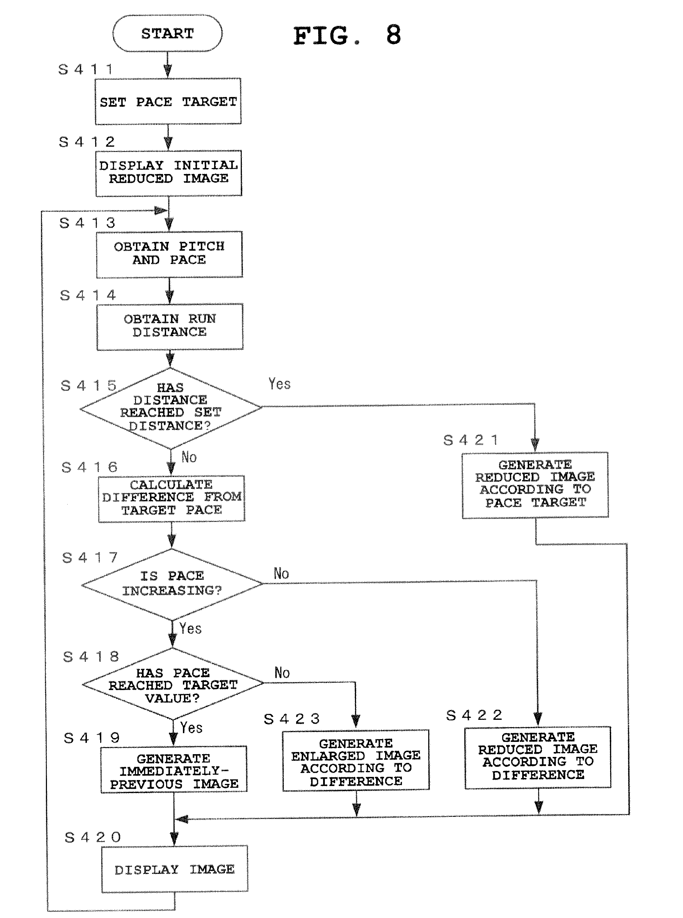

FIG. 8 is a flowchart of an example depicting a build-up mode applied to the exercise support method according to the present embodiment.

Here, description of processing operations similar to those of the respective motion modes described above is simplified.

The build-up mode is a motion mode in which build-up running at a running speed set in advance is promoted to be performed by the virtual person VR.

Here, the build-up running is a way of running in which the running speed is gradually increased for each running distance set in advance, which has been known as a practice method capable of improving endurance and increasing the speed.

The build-up mode applied to the present embodiment is achieved by the user US operating the operation section 130 of the display glasses 100 to select and set the build-up mode as a motion mode, whereby the CPU 140 calls a program module of the control program regarding the build-up mode to perform processing.

In the build-up mode, the user US first operates the operation section 130 of the display glasses 100 to input and set a target value of the running speed (pace), as depicted in FIG. 8 (Step S411). Here, the target value of the running speed to be inputted and set is set so that the running speed is gradually increased for each set distance such as an arbitrary running distance, section distance, etc.

Next, the CPU 140 causes the image generating section 143 and the display driving section 144 to cause a moving image of the virtual person VR registered in advance as an initial image to be displayed in a predetermined area of the display section 110 (Step S412).

Here, as with the case described in the above-described pace-set mode, the initial image is generated as a moving image whose display size has been reduced to achieve a state where the virtual person VR is running in front of the user US in an area relatively away from the user US.

Next, the CPU 140 starts an operation of controlling the motion sensor section 120 to detect at least the acceleration and the angular velocity of the user US during an exercise (during running) by the acceleration sensor 121 and the gyro sensor 122 and output acceleration data and angular velocity data.

Next, the CPU 140 causes the sensor data obtaining section 141 to obtain the acceleration data from the acceleration sensor 121 and the angular velocity data from the gyro sensor 122. The CPU 140 then causes the motion information obtaining section 142 to obtain motion information such as the pitch and the landing/takeoff timing of the feet of the user US, an absolute value of the running speed (pace), and a run distance based on the acceleration data, the angular velocity data, and the running time (Step S413 and Step S414).

Next, the CPU 140 judges at Step S411 whether the obtained run distance has reached a set distance with a target value of the running speed set in advance (Step S415).

When the obtained run distance has not reached the set distance at Step S415, the CPU 140 calculates a difference between the obtained running speed and the target value (target pace) of the running speed set to the set distance (the obtained value-the target value) (Step S416).

The CPU 140 then judges based on the calculated difference whether the obtained running speed is increasing (an absolute value of the difference is reducing) (Step S417).

When judged at Step S417 that the running speed is increasing (the absolute value of the difference is reducing), the CPU 140 judges whether the running speed has reached the target value (Step S418).

When judged at Step S418 that the running speed has reached the target value, the CPU 140 causes the image generating section 143 to generate a moving image of the virtual person VR whose display size has been enlarged to achieve a state where the virtual person VR is running in an area immediately in front of the user US in the viewing field, match the landing/takeoff timing of the feet of the virtual person VR with the landing/takeoff timing of the feet of the user US and the pitch of the virtual person VR with the pitch of the user US based on the pitch and the landing/takeoff timing of the feet of the user US, and generate a moving image of the virtual person VR in synchronization with the motion status of the user US (Step S419).

Next, the CPU 140 causes the display driving section 144 to enlarge the display size and to cause the moving image of the virtual person VR generated in synchronization with the motion status of the user US to be displayed in a predetermined area of the display section 110 (Step S420).

On the other hand, when judged at Step S415 that the obtained run distance has reached the set distance, the CPU 140 causes the image generating section 143 to increase the pitch of the virtual person VR and generate a moving image whose display size has been reduced such that the virtual person VR is moving away from the user US toward the front of the viewing field (Step S421).

Next, the CPU 140 causes the display driving section 144 to reduce the display size and to cause the moving image of the virtual person VR generated by increasing the pitch to be displayed in a predetermined area of the display section 110 (Step S420).

When judged at Step S417 that the running speed is not increasing (the absolute value of the difference is increasing), the CPU 140 judges that the running speed of the user US is decreasing, and causes the image generating section 143 to generate a moving image of the virtual person VR with its display size reduced in, for example, inverse proportion, according to the magnitude of the absolute value of the difference (Step S422).

Next, the CPU 140 causes the display driving section 144 to cause the moving image of the virtual person VR generated by reducing the display size according to the decrease of the running speed to be displayed in a predetermined area of the display section 110 (Step S420).

When judged at Step S418 that the running speed has not reached the target value, the CPU 140 causes the image generating section 143 to generate a moving image of the virtual person VR with its display size enlarged in, for example, inverse proportion, according to the magnitude of the absolute value of the difference (Step S423).

Next, the CPU 140 causes the display driving section 144 to cause the moving image of the virtual person VR generated by enlarging the display size according to the increase of the running speed to be displayed in a predetermined area of the display section 110 (Step S420).

The CPU 140 then performs this series of processing at predetermined time intervals or repeatedly at all times.

As a result, the display size of the virtual person VR displayed in the front of the viewing field of the user US is set to be small for each arbitrary set distance, whereby the user US recognizes that the virtual person VR is running in front of the user US in an area relatively away from the user US. Then, as his or her running speed (pace) gradually becomes closer to the target value set in advance so as to gradually increase for each set distance, the display size of the virtual person VR is set larger. Consequently, the user US is gradually catches up with the virtual person VR.

That is, by adjusting the running speed (pace) so that the user US can catch up with the virtual person VR running ahead or the distance from the virtual person VR is kept constant, the user US can be drawn (the running is guided) by the virtual person VR at the running speed of the target value set in advance for each set distance, which contributes to an improvement of the physical capability of the user US.

### (Interval Mode)

An interval mode applied to the exercise support method according to the present embodiment is achieved by a processing operation approximately similar to that of the build-up mode described above.

Here, with reference to the flowchart (FIG. 8) depicting the above-described build-up mode as appropriate, a processing operation unique to the interval mode according to the present embodiment is described in detail.

The interval mode is a motion mode in which interval running at a running speed set in advance is promoted to be performed by the virtual person VR.

Here, the interval running is a way of running in which a period during which the running speed is increased for running fast and a period during which the running speed is decreased for running slowly are alternately repeated for each running distance set in advance, which has been known as a practice method capable of improving endurance.

The interval mode applied to the present embodiment is achieved by the user US operating the operation section 130 of the display glasses 100 to select and set the interval mode as a motion mode, whereby the CPU 140 calls a program module of the control program regarding the interval mode to perform processing.

In the interval mode, in the flowchart for the build-up mode depicted in FIG. 8, the user US first operates the operation section 130 of the display glasses 100 to input and set a target value of the running speed (pace) (Step S411).

Here, in the interval mode, the target value of the running speed to be inputted and set is set so that a period during which the running speed is increased and a period during which the running speed is decreased are alternately provided for each arbitrary set distance.

Next, the CPU 140 causes a moving image of the virtual person VR as an initial image to be displayed on the display section 110 such that the virtual person VR is running in front of the user US in an area relatively away from the user US (Step S412).

Then, as with the build-up mode described above, the CPU 140 performs this series of processing at Step S413 to Step S420 at predetermined time intervals or repeatedly at all times.

As a result, in the interval mode as well, the user US recognizes that the virtual person VR is running in front of the user US in an area relatively away from the user US for each arbitrary set distance, as in the case of the build-up mode described above. Then, as his or her running speed (pace) becomes closer to the target value set in advance so as to increase or decrease for each set distance, the user US gradually catches up with the virtual person VR.

That is, by adjusting the running speed so that the user US can catch up with the virtual person VR running ahead or the distance from the virtual person VR is kept constant, the user US can be drawn (the running is guided) by the virtual person VR at the running speed of the target value set in advance for each set distance, which contributes to an improvement of the physical capability of the user US.

In the above-described build-up mode and interval mode, the processing operation is described in which the user US sets the running speed (pace) that serves as a target value in advance for each arbitrary set distance and achieves the running that is approximate to this target value. However, the present invention is not limited thereto.

That is, in the above-described build-up mode or interval mode, control may be performed by which, in addition to the running speed (pace), an arbitrary pitch is set as a target value and this pitch is increased and decreased to correspond to the display size (that is, a distance from the user US) of the virtual person VR.

In each of the above-described motion mode, the pitch of the virtual person VR is matched with the pitch of the user US who is a runner so as to extend the stride (footstep width) of the user US to increase the pace. However, the present invention is not limited to this scheme.

For example, a configuration may be adopted in which, when it is judged that the pitch or the pace of the user US is decreasing, or at arbitrary timing, a moving image whose time period from the time of takeoff of the feet of the virtual person VR to the time of landing thereof is slightly shorter than the pitch of the user US, or in other words, a moving image whose pitch is slightly fast is generated and displayed, whereby the pitch of the user US is prompted to increase.

In this case, the pitch to be set to the virtual person may be a pitch that is set so as to increase the pitch of the user US uniformly by a predetermined footstep count, a pitch that is set so as to increase according to the pitch of the user US by, for example, a footstep count at a predetermined ratio, or a pitch that is set variably based on another arithmetic expression, conditional expression, or the like.

Also, in each of the above-described motion mode, a configuration may be adopted in which, when the user US temporarily suspends a running motion to takes a rest, water, or the like during an exercise (during running) and the obtained running speed is decreased (the pace is decreased), the image of the virtual person VR is temporarily stopped by, for example, the user US operating the operation section 130, so that the display operation in which the virtual person VR increases the running speed (increases the pace) and moves away from the user US based on the processing operation described above is not performed.

As described above, with the exercise support apparatus and the exercise support method according to the present embodiment, motion information (pitch, pace, or the like) of the user who is an actual runner is fed back to the generation of an image of the virtual person for display in the viewing field of the user, whereby a function of drawing the user (guiding the running) and a function of sufficiently drawing user's capability (physical capability) can be achieved, like an actual pacemaker.

Conventionally, it is very difficult to secure a human resource satisfying capabilities and conditions required for a pacemaker who is a runner having a running ability equivalent to or more than that of the user himself or herself and is capable of understanding the proficiency of the user and appropriately guiding the user. However, by applying the above-described exercise support apparatus and exercise support method of the present invention, it is possible to easily use a pacemaker optimal to the user himself or herself.

Also, a practice of guiding and drawing the running of the user while adjusting the running speed (pace) does not always work well depending on the physical conditions of the pacemaker. However, with the present invention, it can always be favorably conducted, which contributes to an improvement of the physical capability of the user.

### <Second Embodiment>

Next, a second embodiment of the exercise support apparatus according to the present invention is described.

In the above-described first embodiment, the display section, the motion sensor section, the CPU, etc., are integrally incorporated in the display glasses as a single device.

However, the second embodiment has a structure where at least the display function and the sensor function are separately provided to different devices.

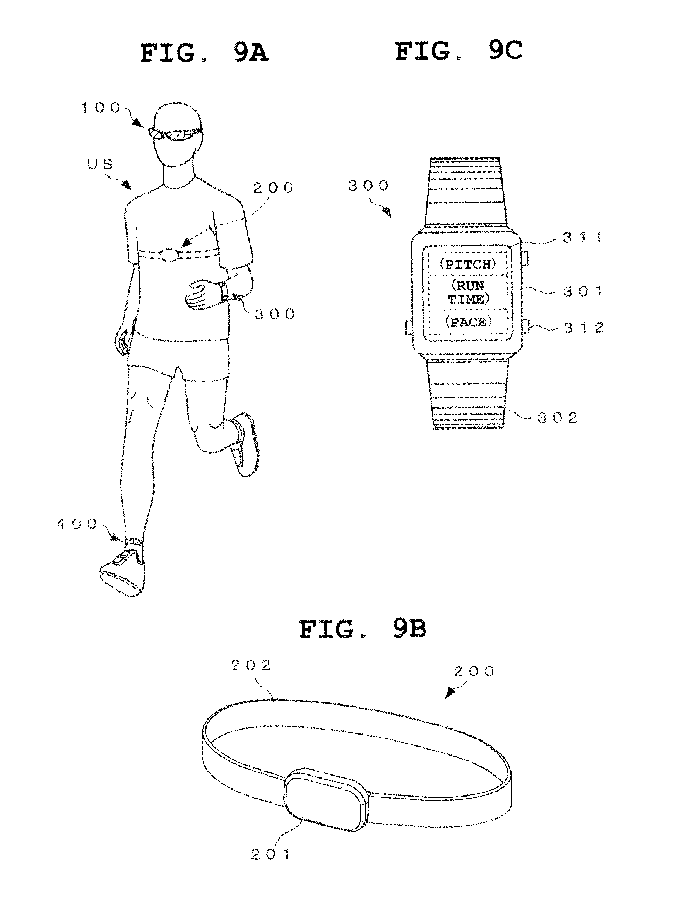

FIG. 9A, FIG. 9B, and FIG. 9C are schematic structural views of the second embodiment of the exercise support apparatus according to the present invention.

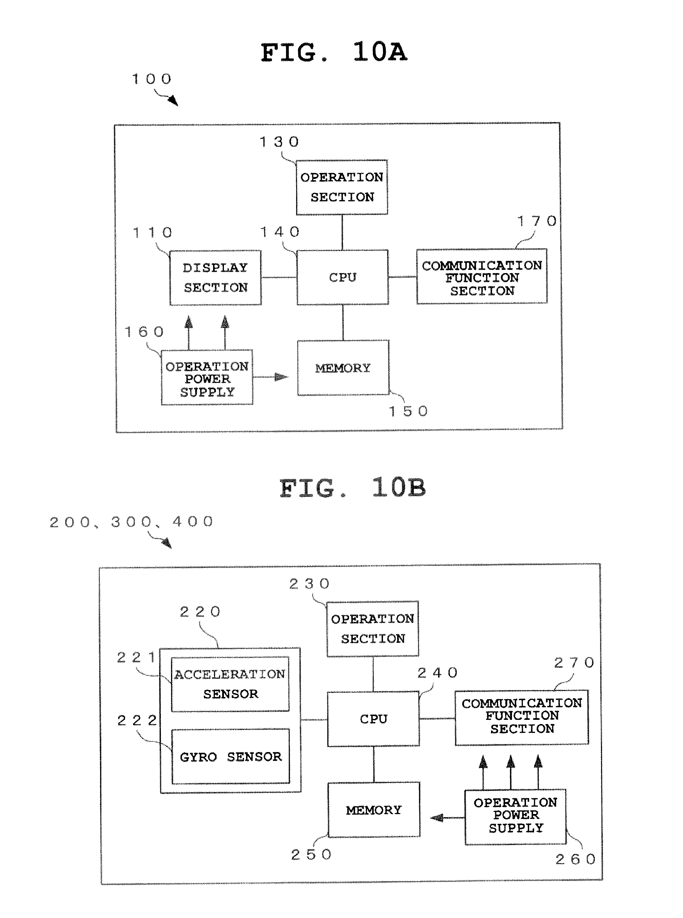

FIG. 10A and FIG 10B are block diagrams showing an example of the structure of a device applied to the exercise support apparatus according to the present embodiment. Here, components similar to those of the above-described first embodiment are provided with the same reference numerals and therefore description thereof is simplified.

The exercise support apparatus according to the second embodiment mainly includes, in addition to the display glasses 100 having a display function, at least one sensor device among a chest sensor 200, a wrist analyzer 300, a foot sensor 400, etc. having a sensor function, as depicted in FIG. 9A.

As with the above-described first embodiment (refer to FIG. 1B and FIG. 1C), the display glasses 100 applied to the present embodiment has an outer appearance of eyeglasses-type or goggles-type, and is mounted on the head part of the user US.

Specifically, the display glasses 100 mainly includes the display section 110, the operation section 130, the CPU 140, the memory 150, the operation power supply 160, and a communication function section 170, as depicted in FIG. 10A.

That is, the display glasses 100 according to the present embodiment has a structure where the motion sensor section 120 is omitted and the communication function section 170 is added in the structure described in the above-described first embodiment (refer to FIG. 2).

Here, the display section 110, the operation section 130, the CPU 140, the memory 150, and the operation power supply 160 have structures and functions approximately similar to those of the first embodiment, and therefore are not described herein.

The communication function section 170 applied to the display glasses 100 transmits data to the sensor devices, such as the chest sensor 200, the wrist analyzer 300, and the foot sensor 400, which will be described further below, by various wireless communication schemes or by a wired communication scheme via a communication cable.

Here, in data transmission by a wireless communication scheme between the display glasses 100 and any one of the sensor devices, for example, Bluetooth (registered trademark), which is a short-range wireless communication standard for digital devices, or Bluetooth (registered trademark) low energy (LE) laid out in this communication standard as a standard of a low power consumption type can be favorably applied. According to the wireless communication scheme, data transmission can be favorably performed even with small electric power generated by the above-described energy harvest technology or the like.

The chest sensor 200 applied to the present embodiment has an outer appearance of a chest sensor, as depicted in FIG. 9B, and mainly includes a device main body 201 having a sensor function and a belt section 202 to be wound around the chest part of the user US to mount the device main body 201 on the chest part.

The wrist analyzer 300 applied to the present embodiment has an outer appearance of a wrist band or a wristwatch, as depicted in FIG. 9C, and mainly includes a device main body 301 having a sensor function and a belt section 302 to be wound around the wrist of the user US to mount the device main body 301 on the wrist.

In FIG. 9C, the structure is depicted where the device main body 301 includes a display section. However, in the present embodiment, a display section is not necessarily required to be provided.

In the structure where a display section has been provided, exercise information such as a pitch, a running speed, a running distance, and a calorie consumption amount are displayed as appropriate, as depicted in FIG. 9C.

The foot sensor 400 applied to the present embodiment has a senor function, and is mounted on an ankle, a shoelace, a shoe sole, or the like of the user US.

Specifically, the sensor device applied to the present embodiment (such as the chest sensor 200, the wrist analyzer 300, and the foot sensor 400) mainly includes a motion sensor section 220, an operation section 230, a CPU 240, a memory 250, an operation power supply 260, and a communication function section 270, as depicted in FIG. 10B.

The motion sensor section 220 has at least one of an acceleration sensor 221, a gyro sensor 222, and a pressure sensor.

Here, the motion sensor section 220, the operation section 230, the CPU 240, the memory 250, and the operation power supply 260 have functions approximately similar to those of the motion sensor section 120, the operation section 130, the CPU 140, the memory 150, and the operation power supply 160 described in the above-described first embodiment, and therefore are not described herein.

The communication function section 270 applied to the sensor device transmits various data to the above-described display glasses 100 by a predetermined communication scheme.

Here, data transmission between the display glasses 100 and the sensor device may be performed at predetermined time intervals in synchronization with the timing of detection by each sensor of the motion sensor section 220 or the timing of performing the processing operation in the above-described exercise support method, or may be performed continuously.

As such, in the present embodiment, the display glasses 100 having the display function and the sensor device having the sensor function (such as the chest sensor 200, the wrist analyzer 300, and the foot sensor 400) are structured to be separated to different devices and both transmit data by a predetermined communication scheme such as a wireless one.

As a result, the sensor device having the sensor function can be mounted on any part without the limitation of the mount position of the display glasses 100 having the display function. Therefore, the structure of the display glasses can be simplified to reduce the weight thereof, whereby a factor that prevents the exercise (running) of the user can be eliminated.

In a case where the exercise support method described in the above-described first embodiment is performed in the present embodiment, the entire series of processing operations may be performed by the CPU 140 provided to the display glasses 100 or may be performed by the CPU 240 provided to the sensor device.

Alternatively, the series of processing operations may be split to be performed by the CPU 140 and the CPU 240.

That is, the functions of the CPU 140 described in the above-described first embodiment (a sensor data obtaining function by the sensor data obtaining section 141, a motion information obtaining function by the motion information obtaining section 142, an image generating function by the image generating section 143, and a display driving function by the display driving section 144) may be divided as appropriate between the CPU 140 of the display glasses 100 and the CPU 240 of the sensor device and performed thereby.

In the present embodiment, the chest sensor 200 mounted on the chest part of the user, the wrist analyzer 300 mounted on the wrist, the foot sensor 400 mounted on an ankle, a shoelace, a shoe sole, or the like have been described as the sensor devices having a sensor function. However, the present invention is not limited thereto, and a sensor device that is mounted on the waist part, the upper arm part, the neck part, or the like may be adopted.

### <Third Embodiment>

Next, a third embodiment of the exercise support apparatus according to the present invention is described.

In the above-described second embodiment, the apparatus has a structure in which the display function and the sensor function are separately provided to different devices.

However, in the third embodiment, the apparatus has a structure where a display function, a sensor function, and an arithmetic processing function are separately provided to different devices.

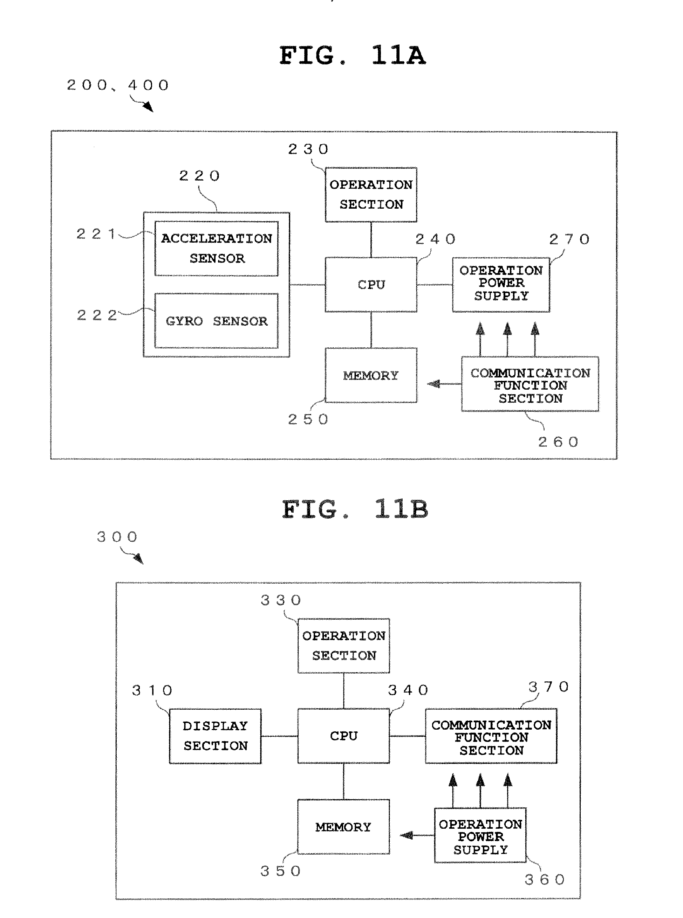

FIG. 11A and FIG 11B are block diagrams showing an example of the structure of a device applied to the exercise support apparatus according to the third embodiment.

Here, components similar to those of the above-described first and second embodiments are provided with the same reference numerals and therefore description thereof is simplified.

As with the above-described second embodiment, the exercise support apparatus according to the third embodiment mainly includes, in addition to the display glasses 100 having the display function, a sensor device having a sensor function such as the chest sensor 200 or the foot sensor 400, and an information processing device having an arithmetic processing function such as the wrist analyzer 300, as depicted in FIG. 9A.

Here, the display glasses 100, the chest sensor 200, and the foot sensor 400 have structures and functions approximately similar to those of the above-described second embodiment, as depicted in FIG. 10A and FIG. 11A, and therefore are not described herein.

Specifically, the information processing device (such as the wrist analyzer 300) applied to the present embodiment mainly includes a display section 310, an operation section 330, a CPU 340, a memory 350, an operation power supply 360, and a communication function section 370, as depicted in FIG. 11B.

On the display section 310, for example, exercise information, such as a pitch, a running speed, a running distance, and a calorie consumption amount, are displayed as appropriate, as depicted in FIG. 9C.

Here, the operation section 330, the CPU 340, the memory 350, the operation power supply 360, and the communication function section 370 have structures and functions approximately similar to those of the operation sections 130 and 230, the CPUs 140 and 240, the memories 150 and 250, the operating power supplies 160 and 260, and the communication function sections 170 and 270 described in the above-described second embodiment, respectively, and therefore are not described herein.

In the present embodiment, the entire series of processing operations of the exercise support method described in the above-described first embodiment is performed by the CPU 340 provided to the wrist analyzer 300.

That is, the chest sensor 200 and the foot sensor 400 serving as sensor devices detect a motion of the user US during an exercise and perform only an operation of outputting corresponding sensor data.

Then, based on the sensor data transmitted from the sensor devices, the wrist analyzer 300 performs the series of processing of the exercise support method described above to generate an image of the virtual person VR whose pitch, display size, and the like have been set, and transmits the image to the display glasses 100. Then, the display glasses 100 performs only an operation of displaying the generated image (moving image) of the virtual person VR on the display section 110.

In the present embodiment, the series of processing operations of the exercise support method is performed only by the CPU 340 provided to the wrist analyzer 300. However, the present invention is not limited thereto.

For example, the series of processing operations may be split to be performed by the CPU 140 provided to the display glasses 100 and the CPU 340.

That is, the functions of the CPU 140 described in the above-described first embodiment (the sensor data obtaining function by the sensor data obtaining section 141, the motion information obtaining function by the motion information obtaining section 142, the image generating function by the image generating section 143, and the display driving function by the display driving section 144) may be divided as appropriate between the CPU 140 of the display glasses 100 and the CPU 340 of the wrist analyzer 300 and performed thereby.

As such, in the present embodiment, the display glasses 100 having the display function, the sensor device having the sensor function (such as the chest sensor 200 and the foot sensor 400), and the information processing device having the arithmetic processing function (such as the wrist analyzer 300) are structured to be separated from each other into different devices and transmit data to and from each other by a predetermined communication scheme such as a wireless scheme.

As a result, the function of each device can be specialized, whereby the sensor device having the sensor function and the information processing device having the arithmetic processing function can be mounted on any part without the limitation of the mount position of the display glasses 100 having the display function.

## Claims

1. An exercise support apparatus (100) comprising:
a sensor section (120, 220) which outputs motion data corresponding to a motion status of a user performing an exercise by moving;
a motion information obtaining section (142) which obtains motion information of the user based on the motion data;
an image generating section (143) which generates a moving image of a virtual person in a moving state, and sets a way of movements of feet of the virtual person to a way of movements corresponding to the obtained motion information of the user; and
a display section (110) which displays the moving image on a part of a display area arranged in a viewing field of the user.

2. The exercise support apparatus (100) according to claim 1, wherein the display area of the display section (110) is a see-through type.

3. The exercise support apparatus (100) according to claim 1, wherein the motion information obtaining section (142) obtains landing and takeoff timing of feet of the user as the motion information, which serves as first timing, and
wherein the image generating section (143) synchronizes motion timing of the feet of the virtual person when landing and taking off with the first timing.

4. The exercise support apparatus (100) according to claim 3, wherein the motion information obtaining section (142) obtains a footstep count of the user per unit time as the motion information, which serves as a first footstep count, and
wherein the image generating section (143) has a period in which landing and takeoff timing of feet of the virtual person has been set to second timing preceding the first timing and footstep count per unit time of the virtual person has been set to a second footstep count larger than the first footstep count.

5. The exercise support apparatus (100) according to claim 3, wherein the sensor section (120, 220) includes an acceleration sensor (121, 221) which detects at least acceleration corresponding to a change ratio of a motion speed of the user and outputs acceleration data corresponding to the acceleration as the motion data, and
wherein the motion information obtaining section (142) obtains the first timing based on the acceleration data.

6. The exercise support apparatus (100) according to claim 5, wherein the motion information obtaining section (142) obtains a plurality of landing and takeoff timings of the feet of the user based on acceleration data outputted from the acceleration sensor (121, 221), and obtains an average value of the plurality of landing and takeoff timings as the first timing.

7. The exercise support apparatus (100) according to claim 1, wherein the sensor section (120, 220) includes an acceleration sensor (121, 221) which outputs acceleration data corresponding to a change ratio of a moving speed of the user and an angular velocity sensor (122, 222) which outputs angular velocity data corresponding to a change of a moving direction of the user,
wherein the motion information obtaining section (142) obtains a value of the moving speed of the user as the motion information, and
wherein the image generating section (143) sets a display size of the virtual person to a size according to the value of the moving speed.

8. The exercise support apparatus (100) according to claim 7, wherein the motion information obtaining section (142) obtains a change amount of the moving speed of the user as the motion information, and
wherein the image generating section (143) sets the display size of the virtual person to be enlarged or reduced according to the change amount of the moving speed obtained by the motion information obtaining section (142).

9. The exercise support apparatus (100) according to claim 7, wherein a target value of a moving speed of the user is set in advance, and
wherein the image generating section (143) sets the display size of the virtual person to be enlarged or reduced according to a difference between the moving speed and the target value.

10. The exercise support apparatus (100) according to claim 7, wherein a target value of a moving speed is set in advance for each moving distance of the user, and
wherein the image generating section (143) sets the display size of the virtual person to be enlarged or reduced according to a difference between the moving speed and the target value for each moving distance of the user.

11. The exercise support apparatus (100) according to claim 1, wherein the display section (110) is positioned in an area in which a lens of an eyeglasses-type device or goggles-type device is positioned, wherein the eyeglasses-type device and the goggles-type device are mounted on a head part of the user.

12. The exercise support apparatus (100) according to claim 1, wherein the sensor section (120, 220), the motion information obtaining section (142), the image generating section (143), and the display section (110) are provided in a single device.

13. The exercise support apparatus (100) according to claim 1, wherein the sensor section (120, 220) and the display section (110) are individually provided in separate devices.

14. An exercise support method for an exercise support apparatus (100) including a display section (110) having a display area that is arranged in a viewing field of a user, comprising:
a step of obtaining motion information of the user based on motion data corresponding to a motion status of the user performing an exercise by moving;
a step of generating a moving image of a virtual person in a moving state;
a step of setting a way of movements of feet of the virtual person to a way of movements corresponding to the obtained motion information of the user; and
a step of displaying the moving image on a part of the display area of the display section (110).

15. The exercise support method according to claim 14, further comprising:
a step of obtaining landing and takeoff timing of feet of the user as the motion information of the user; and
a step of synchronizing motion timing of the feet of the virtual person when landing and taking off with the landing and takeoff timing.
